# EUROPÄISCHE PATENTANMELDUNG

(11) **EP 0 720 854 A1**
(43) Veröffentlichungstag der Anmeldung: **10.07.1996**
(21) Anmeldenummer: 95810816.9
(22) Anmeldetag: 22.12.1995
(51) Int. Cl.: A61K 31/575

(54) **Verwendung von Gallensäuren zur Herstellung von Arzneimitteln zur Behandlung von Erkrankungen der Haut**

(30) Priorität: 04.01.1995 CH 14/95
(71) Anmelder: MEDICHEMIE AG, CH-4107 Ettingen/Basel (CH)
(72) Erfinder: Widauer, Josef Olav, CH-4123 Allschwil (CH)
(74) Vertreter: Hug Interlizenz AG

(57) **Zusammenfassung**

Chenodeoxycholsäure und Ursodeoxycholsaure, beides natürliche Gallensäuren, sind zur Behandlung von Cholestrerin-Gallensteinen, von biliärer Dyspepsie sowie von chronischen Hepatophathien (nur Urso) gut bekannt. Gegenstand der vorliegenden Erfindung ist die Verwendung von diesen Säuren oder eine Kombination davon zur Herstellung eines Arzneimittels zur Behandlung von atopischen Dermatitiden.

## Beschreibung

### TECHNISCHES GEBIET

Die vorliegende Erfindung betrifft die Verwendung der Gallensäuren Chenodeoxycholsäure und Ursodeoxycholsäure oder einer Kombination davon zur Herstellung eines Arzneimittels.

Die chemische Bezeichnung der Chenodeoxycholsäure ist 3α, 7α-Dihydroxy-β-cholan-24-säure. Die Summenformel lautet C24H4004. Die Strukturformel ist

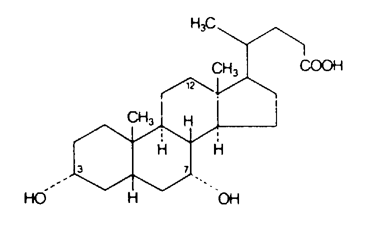

Die chemische Bezeichnung der Ursodeoxycholsäure ist 3α, 7α-Dihydroxy-5β-cholan-24-säure. Die Summenformel lautet C24H4004. Die Strukturformel ist

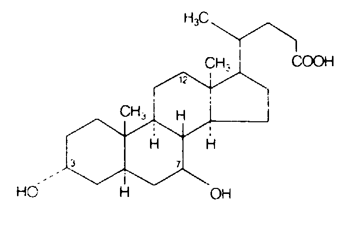

Beide Gallensäuren lassen sich auf halbsynthethischem Wege aus Rindergalle gewinnen.

### STAND DER TECHNIK

Es ist bekannt, die vorgenannten Gallensäuren in Tagesdosen zwischen 5 - 20 mg/kg Körpergewicht zur Auflösung von Cholesterin-Gallensteinen sowie bei biliärer Dyspepsie zu verabreichen. Dabei kommen die genannten Gallensäuren entweder einzeln oder in Kombination miteinander zur Anwendung. Aufgrund etwas unterschiedlicher Wirkungsmechanismen entfalten sie im Mischpräparat einen additiven Effekt. Die Ursodeoxycholsäure wird daneben auch zur Behandlung von Refluxgastritis sowie von chronischen Hepatophatien verwendet. Bezüglich dieser bekannten Indikationen kann z.B. verwiesen werden auf Ulrich Leuschner: "Aktuelle Aspekte der Therapie mit Gallensäuren", Deutsches Ärzteblatt 86, Heft 48, C-2180 - 2186, Nov. 89 oder auf Alan F. Hofmann, "Medical Dissolution of Gallstones by Oral Bile Acid Therapy", The American Journal of Surgery, Volume 158/Number 3, Page 79 - 85, Sept. 89.

### DARSTELLUNG DER ERFINDUNG

Wie nun überraschenderweise gefunden wurde, sind die genannten Gallensäuren auch bei atopischen Dermatitiden therapeutisch gut wirksam. Bekanntlich gehen solche Hauterkrankungen relativ häufig mit Bronchialasthma und/oder vasomotorischer Rhinopathie einher. So wurden Patienten mit schweren Formen atopischer Dermatitis über 4 - 12 Wochen täglich 10 - 30 mg/kg Körpergewicht Ursodeoxycholsäure bzw. Chenodeoxycholsäure, bzw. Placebo, verabreicht. Es ergaben sich bezüglich der diagnostischen Kriterien (Pruritus/Erythem/Infiltration/ Lichenifikation/Schuppung) deutliche Hinweise zugunsten der Behandlung mit den genannten Gallensäuren.

Gegenstand der vorliegenden Erfindung ist demnach die Verwendung von Ursodeoxycholsäure, von Chenodeoxycholsäure bzw. einer Kombination von Urso- und Chenodeoxycholsäure zur Herstellung eines Arzneimittels zur Behandlung von atopischen Dermatitiden.

### Galenische Beispiele

Nachfolgend werden einige galenische Beispiele für die Herstellung von Ursodeoxycholsäure, Chenodeoxycholsäure bzw. Eine Kombination aus beiden enthaltenden Kapseln und Filmtabletten angegeben.

### Beispiel 1

Kapseln
25 kg Ursodeoxycholsäure werden mit Mais- oder Kartoffelstärke, hochdispersem Siliciumdioxid und Magnesiumstearat gemischt und in 100'000 Hartgelatinekapseln mit einem Gehalt von je 250mg Ursodeoxycholsäure abgefüllt.

### Beispiel 2

Kapseln
25 kg Chenodeoxycholsäure werden mit Mais- oder Kartoffelstärke, hochdispersem Siliciumdioxid und Magnesiumstearat gemischt und in 100'000 Hartgelatinekapseln mit einem Gehalt von je 250mg Ursodeoxycholsäure abgefüllt.

### Beispiel 3

Kapseln
Je 25 kg Chenodeoxycholsäure und 25 kg Ursodeoxycholsäure werden mit Mais- oder Kartoffelstärke, hochdispersem Siliciumdioxid und Magnesiumstearat gemischt und in 100'000 Hartgelatinekapseln mit einem Gehalt von je 250mg Chenodeoxycholsäure und 250mg Ursodeoxycholsäure abgefüllt.

### Beispiel 4

Filmtabletten
25 kg Ursodeoxycholsäure werden mit Mais- oder Kartoffelstärke, hochdispersem Silicumdioxid, Milchzucker, Zellulosepulver, Magnesiumstearat und Talk zu einem Granulat verarbeitet und zu Tabletten mit einem Gehalt von je 250 mg Ursodeoxycholsäure gepresst. Diese Tabletten werden mit einem Film überzogen der aus Polymeren wie Zellulosederivaten, Polymethacrylsäureester, Polyvinylpyrrolidon, Polyethylenglykol besteht.

### Beispiel 5

Filmtabletten
25 kg Chenodeoxycholsäure werden mit Mais- oder Kartoffelstärke, hochdispersem Siliciumdioxid, Milchzucker, Zellulosepulver, Magnesiumstearat und Talk zu einem Granulat verarbeitet und zu Tabletten mit einem Gehalt von je 250 mg Chenodeoxycholsäure gepresst. Diese Tabletten werden mit einem Film überzogen, der aus Polymeren wie Zellulosederivaten, Polymethacrysäureester, Polivynylpyrrolidon, Polyethylenglykol besteht.

### Beispiel 6

Filmtabletten
je 25 kg Chenodeoxycholsäure und 25 kg Ursodeoxycholsäure werden mit Mais- oder Kartoffelstärke, hochdispersem Silicumdioxid, Milchzucker, Zellulosepulver, Magnesiumstearat und Talk zu einem Granulat verarbeitet und zu Tabletten mit einem Gehalt von je 250 mg Chenodeoxycholsäure und 250 mg Ursodeoxycholsäure gepresst. Diese Tabletten werden mit einem Film überzogen der aus Polymeren wie Zellulosederivaten, Polymethacylsäureester, Polyvinylpyrrolidon, Polyethylenglykol besteht.

In den vorstehenden Beispielen enthalten die Kapseln und die Filmtabletten mit entweder nur Ursodeoxycholsäure oder nur Chenodeoxycholsäure jeweils 250 mg dieses Wirkstoffes. Bei einer Verabreichung dreimal täglich je einer Tablette bzw. Kapsel entspricht dies bei einer normalgewichtigen Person (75 kg Körpergewicht) etwa einer Tagesdosis von 10 mg/kg Körpergewicht von beiden Wirkstoffen.

## Patentansprüche

1. Verwendung von Chenodeoxycholsäure zur Herstellung eines Arzneimittels zur Behandlung von atopischen Dermatitiden.

2. Verwendung von Ursodeoxycholsäure zur Herstellung eines Arzneimittels zur Behandlung von atopischen Dermatitiden.

3. Verwendung einer Kombination von Chenodeoxycholsäure und Ursodeoxycholsäure zur Herstellung eines Arzneimittels zur Behandlung von atopischen Dermatitiden.
